# EUROPEAN PATENT SPECIFICATION

(11) **EP 4 415 603 B1**
(45) Date of publication and mention of the grant of the patent: **11.11.2026**
(21) Application number: 22792795.1
(22) Date of filing: 20.09.2022
(51) Int. Cl.: A61B 5/00, A61B 5/107

(54) **DETERMINING A TISSUE PROPERTY OF A BODY PART**
BESTIMMUNG EINER GEWEBEEIGENSCHAFT EINES KÖRPERTEILS
DÉTERMINATION D'UNE PROPRIÉTÉ DE TISSU D'UNE PARTIE DU CORPS

(30) Priority: 11.10.2021 EP 21201921
(43) Date of publication of application: 21.08.2024
(73) Proprietor: Koninklijke Philips N.V., 5656 AE Eindhoven (NL)
(72) Inventor: DUINEVELD, Paulus Cornelis, 5656 AG Eindhoven (NL); VAN DE VEEN, Egbert, 5656 AG Eindhoven (NL)
(74) Representative: Philips Intellectual Property & Standards
(86) International application number: PCT/EP2022/075999
(87) International publication number: WO 2023/061704

(56) References cited:
- WO-A1-2014/121945
- US-A- 5 860 934
- US-A1- 2013 158 418
- US-B2- 7 316 652

## Description

### FIELD OF THE INVENTION

This disclosure relates to the use of a shell cuff apparatus to determine a tissue property of a body part or a measurement of a physiological characteristic of a subject, and in particular to computer-implemented methods, systems, and computer program products for determining a tissue property of a body part or a measurement of a physiological characteristic of a subject.

### BACKGROUND OF THE INVENTION

Blood pressure (BP) is an important indicator of health in a person/subject. In the US it is estimated that about 30% of the adult population has high blood pressure. Hypertension is a common health problem which has no obvious outward symptoms. Blood pressure generally rises with aging and the risk of becoming hypertensive in later life is considerable. Persistent hypertension is one of the key risk factors for strokes, heart failure and increased mortality. The condition of a subject can be improved by lifestyle changes, healthy dietary choices and medication. Particularly for high-risk patients, continuous 24-hour blood pressure monitoring is very important by means of systems which do not impede ordinary daily life activities. Continuous monitoring of blood pressure can also be useful for patients in a healthcare environment, such as a hospital e.g. in the operating room (OR) or the Intensive Care Unit (ICU).

In some cases, absolute measurements of blood pressure can be obtained, and in other cases relative measurements of blood pressure can be obtained, for example a measurement of a change in blood pressure. In particular, blood pressure can change over short time windows, e.g. in the order of a few minutes, and these changes can be relevant for further medical examination and possibly medical intervention.

There are a number of different techniques available for measuring blood pressure, and/or changes in blood pressure. Some of these techniques measure blood pressure itself, while other techniques measure other physiological characteristics of the subject and use these as surrogates for blood pressure, for example by relating changes or values of the physiological characteristic to changes or values of blood pressure. Some of the techniques for directly measuring blood pressure require invasive access to the arteries of the subject, for example via a catheter, or the use of bulky/inconvenient equipment such as inflatable cuffs. However, some of the physiological characteristics used as surrogates for blood pressure can be measured using simple and/or unobtrusive sensors applied to the body of the subject.

WO 2014/121945 describes a blood pressure measuring system comprising a kinking-proof shell. The blood pressure measurement system is configured to surround a patient's body part and has pressurization means for applying pressure to the body part and a kinking-proof shell that is located between the pressurization means and the body part. A pressure transducer or pressure sensor pad can be provided on an inner surface of the shell. This arrangement is referred to as a "shell cuff" or "shell cuff apparatus", and the arrangement essentially prevents damping of the pulsatile signals by components of the device and/or by air outside of the device, which translates into a high signal quality and enables blood pressure to be measured with similar accuracy to invasive techniques that use a catheter to directly access the blood. This is achieved by the hydraulically optimized contact of the pressure transducer or pressure sensor pad with the body part that is relevant for the measurement.

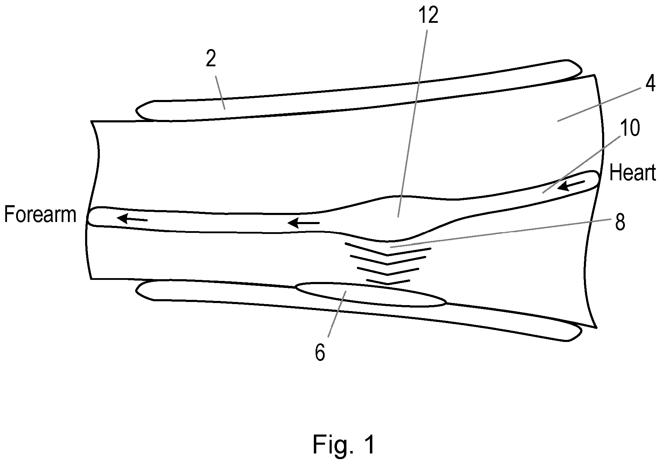

Fig. 1 is a simplified diagram showing a shell cuff apparatus 2 placed at least partially around a body part 4, in this case an arm. The shell cuff apparatus 2 includes a hard shell encased by an inflatable cuff, with a sensor pad 6 arranged on the inside of the hard shell to measure pulse waves 8 passing through the blood vessel 10 (artery) in the body part 4. The pulse wave 8 causes a pressure wave in the tissue in the body part 4 and this is measured by the sensor pad 6.

**In** more detail, a pulse wave 8 in the brachial artery 10 causes a change in the skin movement. This change in skin movement causes a compression of a liquid in the sensor pad 6. The liquid in the sensor pad 6 can be, for example, a silicone oil such as AK10. The sensor pad 6 is connected via a flexible tube filled with a similar liquid as in the sensor pad 6 to a pressure sensor (not shown in Fig. 1) which measures the pressure signal. In the current application the liquid in the pressure sensor is, for compatibility reasons, a different liquid to the liquid in the sensor pad 6. For example, the liquid in the pressure sensor may be glycerol.

The liquid-filled sensor pad 6 is enclosed by a hard shell in the shell cuff apparatus 2 to provide stiffness and cause a sufficient signal to be measured by the pressure sensor. Similar to a standard blood pressure cuff there is an actuator (e.g. an air pump) that can fill a cuff with air, to compress the hard shell and thus the arm tissue, so the brachial artery can be closed.

The blood pressure measurements by the shell cuff apparatus 2 need to be calibrated against blood pressure measurements obtained using an invasive technique (with these measurements being obtained at the same time). This calibration can be done for a large set of patients with different levels of tissue stiffness as the tissue stiffness will influence the transfer of the pressure signal from the artery 10 to the outside of the body part 4. As the shell cuff apparatus 2 includes a hard shell, shell cuffs with different sizes are provided to accommodate patients (subjects) with different arm diameters.

Currently, the effect of individual variation of tissue properties cannot be taken into account in the measurements obtained using a shell cuff apparatus. Instead, a variation of tissue properties is only taken into account according to on average tissue properties expected for a particular size of shell cuff. There can be, however, for a single size of shell cuff, a significant difference in tissue properties between subjects. For instance, subjects with large obese arms will have different tissue properties compared to subjects with muscular arms of similar size.

Thus, it is desirable to be able to determine a tissue property of the body part of the subject, so that this can, for example, be taken into account in any estimates or measurements of physiological characteristics (e.g. blood pressure) that are derived from the measurements obtained using the shell cuff apparatus.

### SUMMARY OF THE INVENTION

The techniques described herein use a sensor to obtain measurements of a radius of the body part, or changes in the radius of the body part, as a number of different pressures are applied to the body part using the shell cuff apparatus. A tissue property of the body part can be determined from a function or relationship between changes in the radius of the body part and the pressures applied by the shell cuff apparatus. The invention is defined by the independent claims. The dependent claims define advantageous embodiments.

According to a first aspect, there is provided a computer-implemented method of determining a tissue property of a body part. The method comprises controlling a shell cuff apparatus to apply a plurality of different pressures to the body part, wherein the shell cuff apparatus at least partially encloses the body part, and wherein the shell cuff apparatus comprises a hard shell portion placed at least partially around the body part and an inflatable cuff surrounding the hard shell portion that is inflated and/or deflated to apply the plurality of different pressures to the body part via the hard shell portion; obtaining radius measurements of a radius of the body part, or changes in the radius of the body part, as the plurality of different pressures are applied to the body part, using a radius sensor or using measurements from a sensor pad positioned on the inside of the hard shell portion; obtaining pressure measurements of the different pressures applied to the body part, wherein the pressure measurements are obtained using the sensor pad. Alternatively, the pressure measurements are obtained using a cuff pressure sensor to obtain measurements of the pressure in the inflatable cuff as the inflatable cuff is inflated and/or deflated and determining the pressure measurements from a respective cuff pressure sensor measurement and a corresponding radius measurement. The method further comprises determining the tissue property of the body part from the radius measurements and the pressure measurements.

According to a second aspect, there is provided a computer-implemented method of determining a measurement of a physiological characteristic of a subject. The method comprises determining a tissue property of a body part of the subject according to the first aspect or any embodiment thereof, using the determined tissue property to calibrate pressure measurements by a sensor pad positioned inside the hard shell portion; and determining a measurement of the physiological characteristic using the calibrated pressure measurements.

According to a third aspect, there is provided a system for determining a tissue property of a body part according to claim 11.

According to a fourth aspect, there is provided a system for determining a measurement of a physiological characteristic of a subject according to claim 12.

According to a fifth aspect, there is provided a computer program product comprising computer readable code, the computer readable code being configured such that, on execution by a system according to the third or fourth aspects, or any embodiment thereof, the system is caused to perform the method according to the first aspect, the second aspect, or any embodiment thereof.

Various embodiments of the system according to the third and fourth aspects are also envisaged in which the system is additionally or further configured to operate according to the various embodiments of the methods described herein.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 is a simplified diagram of a shell cuff apparatus around a body part of a subject;
Fig. 2 is a block diagram of a system for determining a tissue property of a body part that comprises a device and a shell cuff apparatus;
Fig. 3 shows an exemplary hard shell portion with a radius sensor in the form of a strain gauge;
Fig. 4 illustrates a cross-section of a hard shell portion around a simplified model of a body part;
Fig. 5 illustrates a sensor pad being pressed into a body part; and
Fig. 6 is a flow chart illustrating a method of determining a tissue property of a body part.

### DETAILED DESCRIPTION OF EMBODIMENTS

As noted above, the techniques described herein use a sensor to obtain measurements of a radius of the body part, or changes in the radius of the body part, as a number of different pressures are applied to the body part using the shell cuff apparatus. A tissue property of the body part can be determined from a function or relationship between changes in the radius of the body part and the pressures applied by the shell cuff apparatus. The tissue property can be any of: the elastic modulus, the non-linear E-modulus, the Poisson ratio, *vₐᵣₘ,* the fat free mass (FFM), and the adipose fat mass (AFM).

The body part radius or change in body part radius can be measured using a sensor that is referred to herein as a 'radius sensor'. A number of different types of sensors can be used for this purpose. For example, the radius sensor can be or comprise a strain gauge that is either integrated into the hard shell portion of the shell cuff apparatus, or a separate measurement device based on a metal strip, that can be used to measure both the initial body part radius (i.e. before pressure is applied), as well as the change in body part radius as pressure is applied on the body part.

The pressure applied to the body part can be measured directly using a sensor pad associated with the shell cuff apparatus, or indirectly via a measurement of pressure in the inflatable cuff combined with a known friction coefficient between overlapping parts of the hard shell portion, the total length of the hard shell portion and the measured change in body part radius with cuff pressure. The total length of the hard shell portion is a fixed parameter of the hard shell portion, and is length of the shell enclosing the body part (approximately given by *2πR*, with R the body part radius) plus the amount of overlap of the opposite sides of the hard shell portion. A mismatch between both methods being too large is an indication that there is an error in the pressure measured by the sensor pad, which will automatically lead to an error in cardiac output or other physiological characteristic derived from the measured pressure. This can especially occur with small adults with stiff arms. With the proposed techniques, the correct pressure on the body part can be determined, and hence the potential error can be avoided or compensated for.

Thus, embodiments of the techniques enable one or more tissue properties to be determined which can be used for individual calibration of measurements by the sensor pad pressure to determine actual cardiac output, or other physiological characteristics. Further, embodiments provide for the detection of potential errors in measurements of cardiac output or other physiological characteristics, especially for small adults with stiff arms, and correct for these errors.

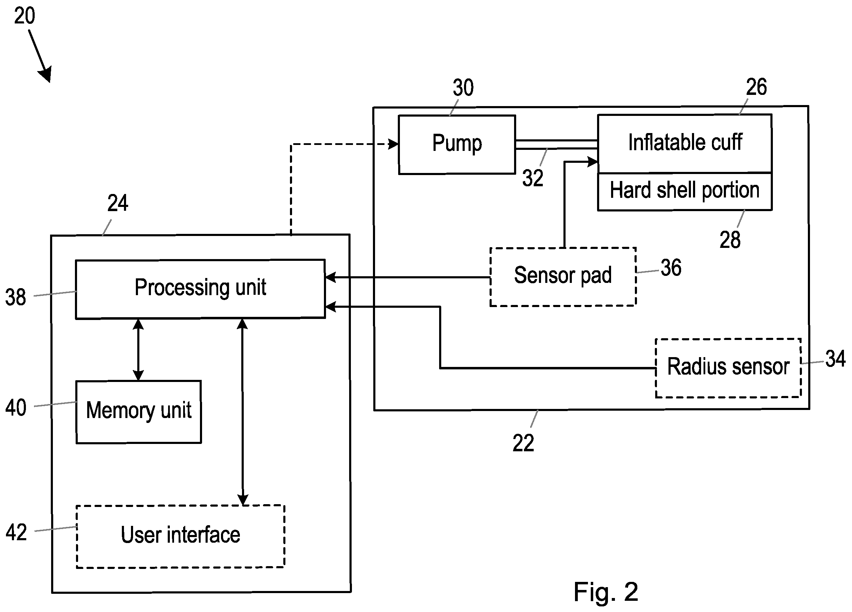

Fig. 2 illustrates an exemplary system 20 for determining a tissue property of a body part. The system 20 comprises a shell cuff apparatus 22 and a device 24. In some embodiments, the system 20 can also be used to determine or measure one or more physiological characteristics of the subject based on the determined tissue property.

The shell cuff apparatus 22 comprises an inflatable cuff 26 and a hard shell portion 28. The hard shell portion 28 is to be placed around at least a portion of a body part, such as an arm or leg, and the inflatable cuff 26 encloses the hard shell portion 28 (i.e. the inflatable cuff 26 is placed around the hard shell portion 28 so that when the inflatable cuff 26 is inflated, it presses the hard shell portion 28 on to the body part). The hard shell portion 28 can be formed from any suitable material, for example a plastic material with an elastic modulus in the range between 0.4 and 5 GPa. Such plastic material can be High Density Polyethylene (HDPE). The length and/or thickness of the hard shell portion 28 can vary with the diameter of the body part that the shell cuff apparatus 22 is to be used on (e.g. the arm or leg). In particular, the larger the diameter of the body part, the larger the length and thickness of the hard shell portion 28. A typical thickness can be in the range between 0.5 and 5 mm, or for example between 1 and 2 mm, with a typical total length can be in the range between 0.25 and 0.7 meters, and for example between 0.3 to 0.5 meters.

The shell cuff apparatus 22 also comprises a pump 30 that is connected to the inflatable cuff 26 (e.g. via a connecting tube 32). The pump 30 is controllable (for example in response to a control signal) to selectively inflate the inflatable cuff 26. The pump 30 may also be able to selectively deflate the inflatable cuff 26, and/or a valve (not shown) may be provided for enabling the inflatable cuff 26 to be selectively deflated.

To enable a tissue property of the body part beneath the inflatable cuff 26 and the hard shell portion 28 to be determined, a radius sensor 34 is provided for measuring the radius of the body part or changes in the radius of the body part as a number of different pressures are applied to the body part by the inflatable cuff 26 and the hard shell portion 28. The radius sensor 34 can take any of a number of different forms, as outlined further below. The radius sensor 34 outputs a measurement signal that comprises measurements of the radius, or changes in the radius, over time.

In some embodiments, for example where the system 20 is to be used for determining a physiological characteristic of the subject that takes into account the determined tissue property, the shell cuff apparatus 22 can further comprise a sensor pad 36 positioned on an inner surface of the hard shell portion 28, i.e. a surface of the hard shell portion 28 that faces the body part when the hard shell portion 28 is placed around the body part. When the inflatable cuff 26 is inflated, the inflatable cuff 26 and the hard shell portion 28 press the sensor pad 36 against the body part to enable the sensor pad 36 to measure the pressure in the body part, for example due to pulse waves passing through the body part. The sensor pad 36 outputs a measurement signal that comprises measurements of the pulse waves or pressure waves over time. The sensor pad 36 may be a liquid-filled pad. The sensor pad 36 can be connected via a flexible tube filled with a similar liquid as in the sensor pad 36 to a pressure sensor outside of the hard shell portion 28 which measures the pressure. The liquid in the sensor pad 36 can be, for example, a silicone oil such as AK10, and the liquid in the pressure sensor can be a different liquid to the liquid in the sensor pad 36, such as glycerol.

In some embodiments, the measurements from the sensor pad 36 can be used to determine the radius of the body part or changes in the radius of the body part, in which case a separate radius sensor 34 is not required.

Measurement signals from the radius sensor 34 and the sensor pad 36 (whichever combination of the radius sensor 34 and the sensor pad 36 are present in the shell cuff apparatus 22) are provided to the device 24 for analysis.

The device 24 operates according to the techniques described herein to determine a tissue property of a body part of a subject. The device 24 is configured to receive the measurement signal from the radius sensor 34 and/or the measurement signal from the sensor pad 36. In some embodiments, the device 24 is configured to control the operation of the pump 30 (for example by sending a control signal to the shell cuff apparatus 22/pump 30), and thereby initiate the inflation of the inflatable cuff 26 at an appropriate time.

The device 24 may be in the form of, or be part of, a computing device, such as a server, desktop computer, laptop, tablet computer, smartphone, smartwatch, etc., or other types of device typically found in clinical environments, such as a patient monitoring device (e.g. a monitoring device located at the bedside of a patient in a clinical environment) that is used to monitor (and optionally display) various physiological characteristics of a subject/patient, including blood pressure, cardiac output (CO), stroke volume (SV), stroke volume variation (SVV), or pulse contour stroke volume (PCSV).

The device 24 includes a processing unit 38 that controls the operation of the device 24 and that can be configured to execute or perform the methods described herein. The processing unit 38 can be implemented in numerous ways, with software and/or hardware, to perform the various functions described herein. The processing unit 38 may comprise one or more microprocessors or digital signal processors (DSPs) that may be programmed using software or computer program code to perform the required functions and/or to control components of the processing unit 38 to effect the required functions. The processing unit 38 may be implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), field-programmable gate arrays (FPGAs), hardware for implementing a neural network and/or so-called artificial intelligence (AI) hardware accelerators (i.e. a processor(s) or other hardware specifically designed for AI applications that can be used alongside a main processor).

The processing unit 38 is connected to a memory unit 40 that can store data, information and/or signals for use by the processing unit 38 in controlling the operation of the device 24 and/or in executing or performing the methods described herein. For example, the memory unit 40 can store any of: radius measurements, pressure measurements, measurement signals from one or both of a radius sensor 34 and a pressure sensor 36, for subsequent use by the processing unit 38. In some implementations the memory unit 40 stores computer-readable code that can be executed by the processing unit 38 so that the processing unit 38 performs one or more functions, including the methods described herein. In particular embodiments, the program code can be in the form of an application for a smartwatch, smartphone, tablet, laptop or computer. The memory unit 40 can comprise any type of non-transitory machine-readable medium, such as cache or system memory including volatile and non-volatile computer memory such as random access memory (RAM), static RAM (SRAM), dynamic RAM (DRAM), read-only memory (ROM), programmable ROM (PROM), erasable PROM (EPROM) and electrically erasable PROM (EEPROM), and the memory unit 40 can be implemented in the form of a memory chip, an optical disk (such as a compact disc (CD), a digital versatile disc (DVD) or a Blu-Ray disc), a hard disk, a tape storage solution, or a solid state device, including a memory stick, a solid state drive (SSD), a memory card, etc.

In some embodiments, the device 24 comprises a user interface 42 that includes one or more components that enables a user of device 24 to input information, data and/or commands into the device 24, and/or enables the device 24 to output information or data to the user of the device 24. Information that can be input via the user interface 42 can include any of: gender, age, height, weight, body mass index (BMI), etc. Information that can be output by the user interface 42 can include any of: information about a tissue property, a value of a tissue property, information about a physiological characteristic of the subject, a physiological characteristic value, etc. The user interface 42 can comprise any suitable input component(s), including but not limited to a keyboard, keypad, one or more buttons, switches or dials, a mouse, a track pad, a touchscreen, a stylus, a camera, a microphone, etc., and/or the user interface 42 can comprise any suitable output component(s), including but not limited to a display screen, one or more lights or light elements, one or more loudspeakers, a vibrating element, etc.

It will be appreciated that a practical implementation of a device 24 and/or shell cuff apparatus 22 may include additional components to those shown in Fig. 2. For example, the device 24 may also include a power supply, such as a battery, or components for enabling the device 24 to be connected to a mains power supply. The device 24 may also include interface circuitry for enabling a data connection to and/or data exchange with other devices, including the shell cuff apparatus 22 (for example if the shell cuff apparatus 22 is not directly connected to the device 24), servers, databases and user devices.

**In** some embodiments, the shell cuff apparatus 22 may comprise one or more further sensors for measuring other properties. For example, in embodiments where the radius sensor 34 is a strain gauge, a temperature sensor can also be provided that can be used to measure the temperature of the environment in which the shell cuff apparatus 22 is being used. The measured temperature can be used to correct the measurement of strain by the strain gauge for changes in temperature. Alternatively, multiple strain gauges can be provided that are coupled in a half or full Wheatstone bridge arrangement to automatically correct for changes in temperature.

In another approach, in implementations where the hard shell portion 28 is tapered to better match the shape of the body part (e.g. arm), multiple radius sensors 34 can be provided on different parts of the hard shell portion 28 to measure the radius or change in radius of those parts.

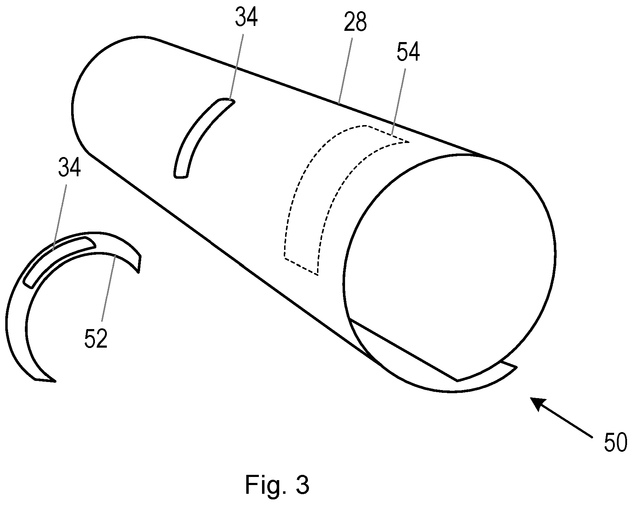

Fig. 3 shows an exemplary hard shell portion 28 with a radius sensor 34 in the form of a strain gauge. The hard shell portion 28 comprises a sheet of material that is curved or 'rolled up' into a cylindrical shape so that it can be placed around a body part. The leading edges of the material overlap in a region 50 as shown in Fig. 3. As the inflatable cuff 26 is inflated, the hard shell portion 28 is compressed against the body part, the amount of overlap in region 50 increases and the diameter of the cylinder formed by the hard shell portion 28 decreases. A radius sensor 34 in the form of a strain gauge can be attached to the outer surface or inner surface of the hard shell portion 28 to measure the strain caused by deformation of the hard shell portion 28. Alternatively, the strain gauge 34 can be integral with the hard shell portion 28. Preferably the hard shell portion 28 is made from a material that has a small coefficient of friction when the material is overlapped. This enables the diameter or size of the hard shell portion 28 change more smoothly as the pressure applied to the outside of the hard shell portion 28 changes.

Fig. 3 also shows an alternative approach which can be used with an existing shell cuff apparatus that does not include a radius sensor 34 to enable the techniques described herein to be used. In this approach, an attachable portion 52 is provided that can be attached to, or placed on, the outer surface or inner surface of a hard shell portion 28, for example at position 54, and that comprises a radius sensor 34 in the form of a strain gauge. Attachment of the attachable portion 52 can be achieved using any suitable attachment means, such as an adhesive, clamp, screw, bolt, etc. Deformation of the hard shell portion 28 when the inflatable cuff 26 is inflated causes corresponding deformation of the attachable portion 52, and the radius sensor 34 measures the strain associated with this deformation. In a particular implementation, the attachable portion 52 is formed from steel that is 0.01mm to 5mm thick. The force (clamping force) with which the attachable portion 52 is attached to the hard shell portion 28 should be small compared to the pressure that can be exerted by the inflatable cuff 26 when it is inflated in order to avoid influencing the pressure in the inflatable cuff 26.

The processing unit 38 or the radius sensor 34 may comprise suitable electronic components or software to enable the measurements of the radius or changes in radius by the radius sensor 34 to be made and/or interpreted. For example, in embodiments where the radius sensor 34 is a strain gauge, the radius sensor 34 or the processing unit 38 can comprise an electrical circuit in the form of a Wheatstone bridge that is used to obtain the measurement from the strain gauge. A low-pass frequency filter may also be provided in the processing unit 38 or the radius sensor 34 for filtering out low frequency noise such as pressure pulses from the pump 30 and/or heart beat pulses. A typical cut-off frequency is preferably lower than 20 Hertz (Hz) and more preferably lower than 0.5 Hz.

As noted above, the radius sensor 34 and can take any of a number of different forms, and a strain gauge arranged as shown in Fig. 3 is one possibility. Other types of radius sensor 34 can be a sensor or sensors for measuring one or more dimensions, or change in dimension, of the hard shell portion 28. Dimensions that could be measured to determine the radius or change in radius include the diameter of the hard shell portion 28, the circumference of the hard shell portion 28, the tangential displacement of the hard shell portion 28 or the amount of overlap of the hard shell portion 28 in region 50. Types of sensors that can be used to measure one or more dimensions include optical sensors (e.g. a camera, imaging unit, or laser-based system, which may or may not require the placement or presence of visual markers on the hard shell portion 28), proximity sensors, pressure (touch) sensors arranged at different positions on the hard shell portion 28 to measure the amount of overlap in region 50, an electrical property sensor (e.g. that measures resistance, conductance, impedance, etc. between two parts of the hard shell portion 28 in the overlap region 50), an accelerometer that measures the movement of the hard shell portion 28 from which the deformation of the hard shell portion 28 can be derived, and a volume sensor that measures a volume of fluid that flows to and/or from the body part beneath the hard shell portion 28 as the inflatable cuff 26 is inflated and deflated (a measured change in volume of the body part is indicative of the change in volume of the hard shell portion 28 surrounding the body part).

In embodiments where a separate radius sensor 34 is not provided and the measurements from the sensor pad 36 are used to determine the radius or changes in radius of the body part, a measurement of the total air (or other fluid) volume in the inflatable cuff 26 and a measurement of the pressure in the inflatable cuff 26 can be combined to give a relationship between the change in radius of the body part and the pressure on the body part. The volume or air (or other fluid) in the inflatable cuff 26 can be measured using a flow meter, with the measurements of air (fluid) flow being integrated over time to give the volume of air (fluid) in the inflatable cuff 26.

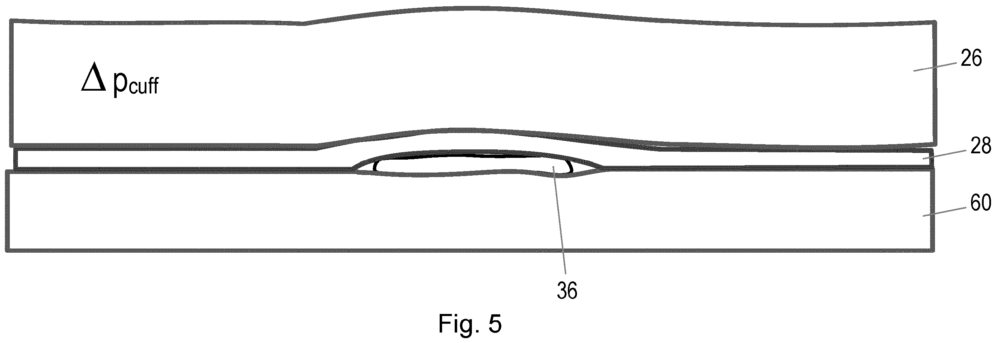

An alternative method to determine the body part radius without a radius sensor 34 is in the case of an overlapping hard shell portion 28. It can be shown that a relation for the body part radius, *R*(Δ*pᵢ*)*,* can be derived as: $R \left(Δ{p}_{i}\right) = \frac{\frac{1}{2} μ \left(1+ε\right) {L}_{t}}{1 - ε + μπ \left(1+ε\right)}$ (1) where ε is the efficacy (a ratio between sensor pad pressure and cuff pressure, which is described further below), µ is a friction factor between the overlapping shell parts and *Lₜ* is the total length of the hard shell portion 28, which is different for different shell cuff sizes. Thus, to determine the body part radius, the efficacy ε and the friction factor µ are required to be known or measured. Although this approach is simple and does not require a separate radius sensor, it is likely to be less accurate as the friction factor µ may vary between overlapping shell parts, and the model that is used to calculate the body part radius is not fully applicable due to the effect described below with reference to Fig. 5.

As described above, it is useful to determine one or more tissue properties of the subject as the response of a pressure sensor (the sensor pad 36) in the hard shell portion 28 is strongly influenced by the tissue properties of a subject. In principle, it is possible to determine, from the change in radius of the body part with the applied pressure on the outside of the body part, a non-linear elastic modulus (E-modulus) of the body part. The details of these relations will be discussed further below, but it can be understood that a relatively large decrease in body part radius with increasing pressure is an indication of a subject with different tissue properties compared to a subject that has a smaller decrease in radius for the same changes in pressure.

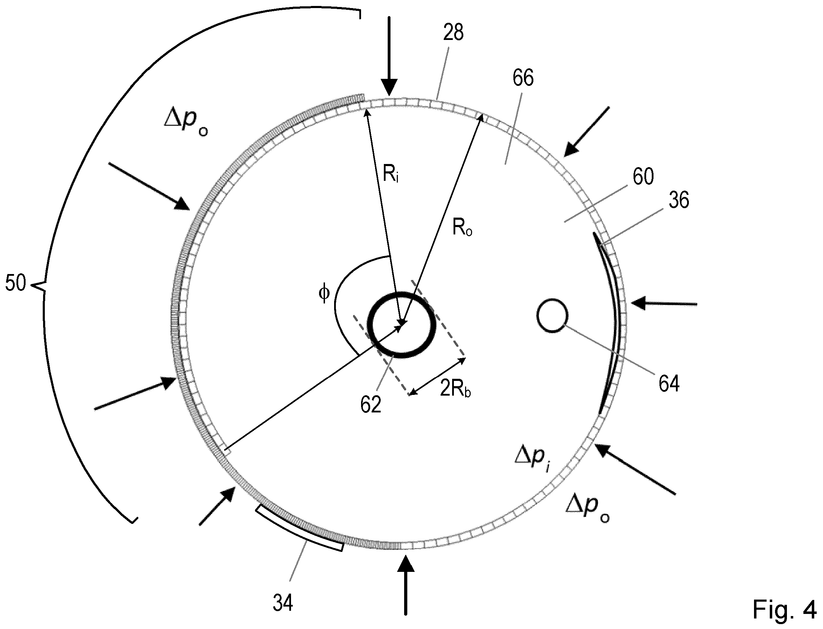

The diagram in Fig. 4 illustrates a cross-section of a hard shell portion 28 around a simplified model of a body part 60. The body part 60 is assumed to have a circular cross-section and includes a bone 62 at the centre of the body part 60 with radius R_{b}, an artery 64 and other soft tissue 66. **In** this model the soft tissue 66 is considered to be uniform (i.e. uniform density and other material properties), but it will be appreciated that more complicated models can be used that includes different tissue sections representing fat, muscle, etc. A radius sensor 34 is provided on the hard shell portion 28, and can be, for example, a strain gauge. A sensor pad 36 is positioned on the inside surface of the hard shell portion 28 and is pressed into the body part 60. The hard shell portion 28 is wrapped around the body part 60 and there is an overlap region 50 where leading edges of the hard shell portion 28 overlap with each other. The amount of overlap (i.e. the length of the shell overlap, *Lₒ*) is given by: ${L}_{o} = {L}_{t} - 2 {πR}_{i} \left(Δ{p}_{i}\right)$ (2) where *Lₜ* is the total length of the hard shell portion 28 in the direction around the body part 60, *Rᵢ*(*Δpᵢ*) is the radius of the body part as a function of pressure applied on the inside of the body part 60 (which is denoted *Δpᵢ*). The total length *Lₜ* of the hard shell portion 28 is a fixed parameter of the hard shell portion 28, measured from one side of the hard shell portion 28 to the other side of the hard shell portion 28. With reference to Fig. 4, the total length *Lₜ* is the full length of the hard shell portion 28 around the body part 60, including the length of the hard shell portion 28 in the overlap region 50. The pressure applied on the inside of the body part 60 is measured by the sensor pad 36. Although not shown in Fig. 4, an inflatable cuff 26 is arranged around the hard shell portion 28 so that when the inflatable cuff 26 is inflated, pressure (as indicated by the arrows directed in towards the hard shell portion 28) is applied to the hard shell portion 28.

The thickness of the hard shell portion 28, t is given by: $t = {R}_{o} - {R}_{i}$ (3) where *Rₒ* is the distance from the centre of the body part 60 to the outer surface of the hard shell portion 28.

An initial radius of the body part, denoted *Rᵢ₀*, can be measured or determined using the radius sensor 34 when no or a very small pressure is being applied to the body part 60 by the inflatable cuff 26.

The radius sensor 34 is used to measure the radius or changes in radius of the body part 60 as the pressure in the inflatable cuff 26 (*Δpₒ*) is varied. The sensor pad 36 is used to measure the pressure applied to the body part 60 (*Δpᵢ*) by the hard shell portion 28. The following equation (4) sets out how one or more tissue properties (namely the elastic modulus, non-linear E-modulus, the Poisson ratio, *vₐᵣₘ,* the fat free mass (FFM), and the adipose fat mass (AFM)) can be derived from the changes in radius Δ*Rᵢ* and the pressure applied to the body part 60: $Δ {R}_{i} = \frac{- 1}{{E}_{arm} \left(Δ{p}_{i}\right)} \frac{{R}_{i 0} \left(1-{υ}_{arm}^{2}\right) \left({R}_{i 0}^{2}-{R}_{b}^{2}\right) {Δp}_{i}}{\left({R}_{i 0}^{2}\left(1+{υ}_{arm}\right)+{R}_{b}^{2}\left(1-{υ}_{arm}\right)\right)}$ (4) with ${E}_{arm} \left(Δ{p}_{i}\right) = {E}_{0} + k ⋅ Δ {p}_{i}$ (5) where *E₀* is the elastic modulus of the tissue 66, *k* is a parameter describing a non-linear E-modulus of the tissue 66, *vₐᵣₘ* is the Poisson ratio of the tissue 66 (assuming the tissue 66 to be a uniform material). The Poisson ratio will be of the order of 0.4 - 0.5, with 0.5 for an incompressible isotropic material deformed elastically at small strains.

It will be appreciated that the relationship shown in equation (4) is based on the simplified body part model in Fig. 4, and different relationships can be derived for more complicated models, e.g. where the body part is considered to be made up of different types of tissue such as muscle and fat.

In the above approach, the pressure on the body part 60 (*Δpᵢ*) is measured using the sensor pad 36. However, in an alternative approach, the pressure on the body part 60 can be determined by measuring the pressure in the inflatable cuff 26 (the "cuff pressure"), which is denoted *Δpₒ.* Due to the properties of the hard shell portion 28, the cuff pressure *Δpₒ* is different (larger) than the pressure *Δpᵢ* on the inside of the hard shell portion 28 which is compressing the body part 60.

A simple analytical relation has been found between the pressure as measured by the sensor pad 36 and the cuff pressure. The ratio between these pressures is called the efficacy ε. The efficacy ε depends on the friction factor, the total length of the hard shell portion *(Lt),* and the actual body part radius (which is a function of the applied pressure). The efficacy ε is given by: $ε = \frac{1 - \frac{1}{2} μ \left(\frac{{L}_{t}}{R \left(εΔ{p}_{o}\right)}-2π\right)}{1 + \frac{1}{2} μ \left(\frac{{L}_{t}}{R \left(εΔ{p}_{o}\right)}-2π\right)}$ (6) Hence with the measured body part radius and the known friction factor and the total length of the hard shell portion 28, the pressure on the inside of the body part can be determined via the cuff pressure. It has been found that a more compressible body part will give lower efficacy. It is possible to obtain an efficacy as low as approximately 0.4 with a friction coefficient (of the hard shell portion 28 material of less than 0.15.

The derivation of the efficacy ε also enables the measurement of the pressure by the sensor pad 36 to be verified. In particular, for smaller sized body parts that produce a relatively small change in the radius on the application of pressure, there is a risk that the pressure measurement by the sensor pad 36 is not the actual pressure acting on the body part. This is illustrated in Fig. 5. For a small, stiff body part, the sensor pad 36 may not be pushed completely into the body part and the pressure in the sensor pad 36 may be larger than the pressure acting on the rest of the body part. Therefore, the use of the efficacy ε enables the measurement of pressure by the sensor pad 36 to be verified based on the measured cuff pressure.

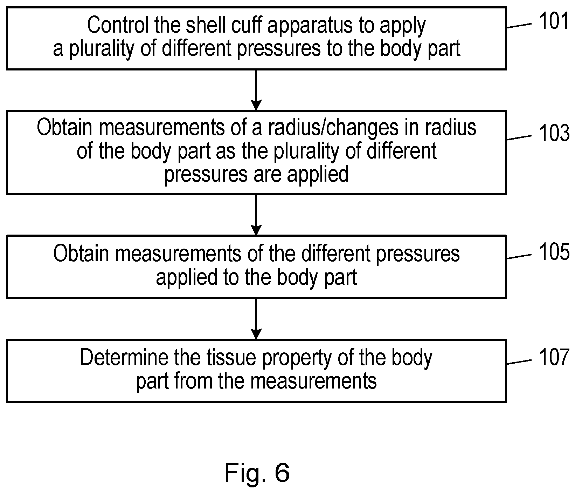

The flow chart in Fig. 6 illustrates a method of determining a tissue property of a body part according to the techniques described herein. The method can be performed by the device 24, and in particular by the processing unit 38. The method can be performed as a result of the device 24 or processing unit 38 executing suitable computer-readable code, that may, for example, be stored in the memory unit 40.

During the measurement stage of the method, represented by steps 101, 103 and 105, a shell cuff apparatus 22 is placed partially or fully around a body part for which one or more tissue properties is to be determined. The shell cuff apparatus 22 comprises a hard shell portion 28 that is placed partially or fully around the body part, and there is an inflatable cuff 26 surrounding the hard shell portion 28. The inflatable cuff 26 can be inflated and/or deflated via a pump 30 to apply multiple different pressures to the body part over time via the hard shell portion 28.

Thus, in a first step, the shell cuff apparatus 22 is controlled to apply a plurality of different pressures to the body part. The shell cuff apparatus 22 can be controlled by the device 24 or processing unit 38, and in particular a pump 30 can be controlled to inflate and/or deflate the inflatable cuff 26. In step 101 the inflatable cuff 26 may be continuously inflated or deflated, or the inflatable cuff 26 may be inflated or deflated in a step-wise manner (i.e. the inflatable cuff 26 is inflated/deflated to a first pressure, the pressure is held for a short period of time, and the inflatable cuff 26 is inflated/deflated to another pressure, and so on). The processing unit 38 can control the application of the plurality of different pressures by the shell cuff apparatus 22 by outputting a suitable control signal to the shell cuff apparatus 22.

In step 103, measurements of a radius of the body part, or measurements of changes in the radius of the body part, are obtained. These measurements are referred to as 'radius measurements'. Radius measurements are obtained as the plurality of different pressures are applied to the body part (i.e. as the inflatable cuff 26 is inflated and/or deflated). That is, multiple radius measurements are obtained, each for a different applied pressure.

In some embodiments, the radius measurements are obtained using one or more radius sensors 34. The radius sensor or sensors 34 can be sensors configured to measure deformation of the hard shell portion 28. Since the hard shell portion 28 is placed around the body part, deformation of the hard shell portion 28 results in a change in radius of the body part beneath the hard shell portion 28. The radius sensor 34 can be configured or arranged such that deformation of the hard shell portion 28 changes one or more electrical properties of the radius sensor 34, such as the resistance, capacitance, inductance. The radius measurements can be derived from changes in these electrical properties. In some embodiments, the radius sensor 34 is a strain gauge.

In alternative embodiments, the radius measurements can be obtained from measurements of the pressure in the inflatable cuff 26, and measurements of the rate of fluid flow into and out of the inflatable cuff 26. In these embodiments, a cuff pressure sensor can be provided for measuring the pressure in the inflatable cuff 26, and a flow meter can be provided for measuring the rate of fluid flow into and/or out of the inflatable cuff 26. Thus, in these alternative embodiments, the radius measurements are obtained by processing the measurements of the pressure in the inflatable cuff 26 and the measurements of the rate of fluid flow into and out of the inflatable cuff 26.

In step 105, measurements of the different pressures applied to the body part are obtained. These measurements are referred to as 'pressure measurements'. The pressure measurements are obtained at the same time as the radius measurements, so that, for each radius measurement, there is a corresponding measurement of the pressure being applied to the body part at that time. This enables the change in radius of the body part to be observed for changes in the applied pressure.

In some embodiments, the pressure measurements are obtained using a sensor pad 36 positioned on the inside of the hard shell portion 28. In alternative embodiments, a cuff pressure sensor can be used to obtain measurements of the pressure in the inflatable cuff 26 as the inflatable cuff 26 is inflated and/or deflated, and pressure measurements can be determined from a respective cuff pressure sensor measurement and a corresponding radius measurement (i.e. a radius measurement obtained at generally the same time as the cuff pressure sensor measurement). In particular, a pressure measurement can be determined from the cuff pressure sensor measurement, the corresponding radius measurement, a friction factor (friction coefficient) of the hard shell portion 28, and the total length of the hard shell portion 28.

Once the measurements have been obtained in steps 103 and 105, in step 107 a tissue property of the body part can be determined from the radius measurements and the pressure measurements. In some embodiments, step 107 comprises using a function that relates changes in radius of the body part to one or more tissue properties and the pressure applied to the body part. The function can be based on a model of the structure of the body part. For example, the model can assume that the tissue property is uniform throughout the body part, or that certain parts of the body part comprise fat and others comprise muscle, each with different tissue properties.

In some embodiments, step 107 comprises evaluating equation (4) to determine any of the tissue properties *E₀, k,* and *vₐᵣₘ*.

In some embodiments, once the tissue property has been determined, the tissue property can be used to calibrate pressure measurements obtained using the shell cuff apparatus 22 for the purpose of determining a physiological characteristic of the subject. The physiological characteristic can be any of blood pressure, cardiac output, stroke volume (SV), stroke volume variation, or pulse contour stroke volume (PCSV). Those skilled in the art will be aware of techniques for calibrating pressure measurements according to a determined tissue property. For example, WO 2019/211210 describes a calibration for SV and PCSV based on the tissue properties FFM and AFM, and the techniques described herein can be used to determine those tissue properties and thereby improve the calibration.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A computer-implemented method of determining a tissue property of a body part, the method comprising:
controlling (101) a shell cuff apparatus (22) to apply a plurality of different pressures to the body part, wherein the shell cuff apparatus at least partially encloses the body part, and wherein the shell cuff apparatus comprises a hard shell portion placed at least partially around the body part, and an inflatable cuff surrounding the hard shell portion that is inflated and/or deflated to apply the plurality of different pressures to the body part via the hard shell portion;
obtaining (103) radius measurements of a radius of the body part, or changes in the radius of the body part, as the plurality of different pressures are applied to the body part, using a radius sensor (34) or using measurements from a sensor pad (36) positioned on the inside of the hard shell portion;
obtaining (105) pressure measurements of the different pressures applied to the body part,
wherein the pressure measurements are obtained using the sensor pad, or
wherein the pressure measurements are obtained using a cuff pressure sensor to obtain measurements of the pressure in the inflatable cuff as the inflatable cuff is inflated and/or deflated and determining the pressure measurements from a respective cuff pressure sensor measurement and a corresponding radius measurement; and
determining (107) the tissue property of the body part from the radius measurements and the pressure measurements.

2. A method as claimed in claim 1, wherein the step of determining the pressure measurements comprises:
determining a pressure measurement as a function of a cuff pressure sensor measurement, a corresponding radius measurement, a coefficient of friction of the hard shell portion, and a total length of the hard shell portion.

3. A method as claimed in claim 1, wherein the radius sensor is a sensor configured to measure deformation of the hard shell portion.

4. A method as claimed in claim 1 or 3, wherein the radius sensor is configured or arranged such that deformation of the hard shell portion changes one or more electrical properties of the radius sensor.

5. A method as claimed in any of claims 1, 3, or 4, wherein the radius sensor is a strain gauge.

6. A method as claimed in claim 1 or 2, wherein the radius measurements are obtained from measurements of the pressure in the inflatable cuff, and measurements of the rate of fluid flow into and out of the inflatable cuff.

7. A method as claimed in claim 1, wherein the radius measurements are obtained from measurements of the pressure in the inflatable cuff, pressure measurements obtained using the sensor pad, a friction factor for the hard shell portion, and the length of the hard shell portion.

8. A method as claimed in any of claims 1-7, wherein the step of determining (107) the tissue property of the body part from the radius measurements and the pressure measurements comprises using a function that relates changes in radius of the body part to one or more tissue properties and the pressure applied to the body part.

9. A method as claimed in any of claims 1-8, wherein the step of determining (107) the tissue property comprises evaluating, using the obtained radius measurements, Δ*Rᵢ*, and the obtained pressure measurements, *Δpᵢ*: $Δ {R}_{i} = \frac{- 1}{{E}_{arm} \left(Δ{p}_{i}\right)} \frac{{R}_{i 0} \left(1-{υ}_{arm}^{2}\right) \left({R}_{i 0}^{2}-{R}_{b}^{2}\right) {Δp}_{i}}{\left({R}_{i 0}^{2}\left(1+{υ}_{arm}\right)+{R}_{b}^{2}\left(1-{υ}_{arm}\right)\right)}$ with ${E}_{arm} \left(Δ{p}_{i}\right) = {E}_{0} + k ⋅ Δ {p}_{i}$ where *E₀* is the elastic modulus of tissue in the body part, *k* is a parameter describing a non-linear E-modulus of the tissue, *vₐᵣₘ* is the Poisson ratio of the tissue, *Rᵢ₀* is an initial radius of the body part when no pressure is applied to the body part by the shell cuff apparatus, and *R_{b}* is a radius of a bone in the body part.

10. A computer-implemented method of determining a measurement of a physiological characteristic of a subject, the method comprising:
determining a tissue property of a body part of the subject according to the method of any of claims 1-9;
using the determined tissue property to calibrate pressure measurements by the sensor pad; and
determining a measurement of the physiological characteristic using the calibrated pressure measurements.

11. A system (20) for determining a tissue property of a body part, the system comprising:
a shell cuff apparatus (22) configured to at least partially enclose a body part and to apply a plurality of different pressures to the body part, wherein the shell cuff apparatus (22) comprises a hard shell portion (28) configured to be placed around at least a portion of the body part and an inflatable cuff (26) surrounding the hard shell portion (28) that is configured to be inflated and/or deflated to apply the plurality of different pressures to the body part via the hard shell portion (28);
one or more sensors (34, 36) for providing radius measurements of a radius of the body part, or changes in the radius of the body part, as the plurality of different pressures are applied to the body part, and pressure measurements of the different pressures applied to the body part, wherein
• to provide the radius measurements, the sensors (34, 36) include a radius sensor (34) or a sensor pad (36) positioned on the inside of the hard shell portion, and
• to provide the pressure measurements, the sensors (34, 36) include the sensor pad (36) or a cuff pressure sensor; and
a device (24) configured to determine the tissue property of the body part, wherein the device comprises a processing unit (38) configured to carry out the method of any of claims 1-9.

12. A system (20) for determining a measurement of a physiological characteristic of a subject, the system comprising:
a shell cuff apparatus (22) configured to at least partially enclose a body part and to apply a plurality of different pressures to the body part, wherein the shell cuff apparatus (22) comprises a hard shell portion (28) configured to be placed around at least a portion of the body part and an inflatable cuff (26) surrounding the hard shell portion (28) that is configured to be inflated and/or deflated to apply the plurality of different pressures to the body part via the hard shell portion (28);
one or more sensors (34, 36) for providing radius measurements of a radius of the body part, or changes in the radius of the body part, as the plurality of different pressures are applied to the body part, and pressure measurements of the different pressures applied to the body part, wherein
• to provide the radius measurements, the sensors (34, 36) include a radius sensor (34) or a sensor pad (36) positioned on the inside of the hard shell portion, and
• to provide the pressure measurements, the sensors (34, 36) include the sensor pad (36) or a cuff pressure sensor; and
a device (24) configured to determine the measurement of the physiological characteristic of the subject, wherein the device comprises a processing unit (38) configured to carry out the method of claim 10.

13. A computer program product comprising instructions to cause the system of claim 11 to execute the method as claimed in any of claims 1-9.

14. A computer program product comprising instructions to cause the system of claim 12 to execute the method as claimed in claim 10.

## Patentansprüche

1. Computerimplementiertes Verfahren zum Bestimmen einer Gewebeeigenschaft eines Körperteils, wobei das Verfahren Folgendes umfasst:
Steuern (101) einer Schalenmanschetteneinrichtung (22), um eine Vielzahl von unterschiedlichen Drücken auf das Körperteil anzuwenden, wobei die Schalenmanschetteneinrichtung das Körperteil mindestens teilweise umschließt, und wobei die Schalenmanschetteneinrichtung einen Hartschalenabschnitt umfasst, der mindestens teilweise um das Körperteil platziert wird, und eine aufblasbare Manschette, die den Hartschalenabschnitt umgibt, die aufgeblasen und/oder entlüftet werden kann, um über den Hartschalenabschnitts die Vielzahl von unterschiedlichen Drücken auf das Körperteil anzuwenden;
Erhalten (103) von Radiusmessungen eines Radius des Körperteils oder von Änderungen des Radius des Körperteils, wenn die Vielzahl von Drücken auf das Körperteil angewendet wird, unter Verwendung eines Radiussensors (34) oder unter Verwendung von Messungen von einem Sensorpad (36), das an der Innenseite des Hartschalenabschnitts positioniert ist;
Erhalten (105) von Druckmessungen der verschiedenen auf das Körperteil angewendeten Drücke,
wobei die Druckmessungen unter Verwendung des Sensorpads erhalten werden, oder
wobei die Druckmessungen unter Verwendung eines Manschettendrucksensors erhalten werden, um Messungen des Drucks in der aufblasbaren Manschette zu erhalten, während die aufblasbare Manschette aufgeblasen und/oder entlüftet wird, und Bestimmen der Druckmessungen aus einer jeweiligen Manschettendrucksensormessung und einer entsprechenden Radiusmessung; und
Bestimmen (107) der Gewebeeigenschaft des Körperteils aus den Radiusmessungen und den Druckmessungen.

2. Verfahren nach Anspruch 1, wobei der Schritt des Bestimmens der Druckmessungen Folgendes umfasst:
Bestimmen einer Druckmessung als Funktion einer Manschettendrucksensormessung, einer entsprechenden Radiusmessung, eines Reibungskoeffizienten des Hartschalenabschnitts und einer Gesamtlänge des Hartschalenabschnitts.

3. Verfahren nach Anspruch 1, wobei der Radiussensor ein Sensor ist, der so konfiguriert ist, dass er Verformung des Hartschalenabschnitts misst.

4. Verfahren nach Anspruch 1 oder 3, wobei der Radiussensor so konfiguriert oder angeordnet ist, dass Verformung des Hartschalenabschnitts eine oder mehrere elektrische Eigenschaften des Radiussensors verändert.

5. Verfahren nach einem der Ansprüche 1, 3 oder 4, wobei der Radiussensor ein Dehnungsmessstreifen ist.

6. Verfahren nach Anspruch 1 oder 2, wobei die Radiusmessungen aus Messungen des Drucks in der aufblasbaren Manschette und Messungen der Fluidstromrate in und aus der aufblasbaren Manschette erhalten werden.

7. Verfahren nach Anspruch 1, wobei die Radiusmessungen aus Messungen des Drucks in der aufblasbaren Manschette, Druckmessungen, die unter Verwendung des Sensorpads erhalten wurden, einem Reibungsfaktor für den Hartschalenabschnitt und der Länge des Hartschalenabschnitts erhalten werden.

8. Verfahren nach einem der Ansprüche 1-7, wobei der Schritt des Bestimmens (107) der Gewebeeigenschaft des Körperteils aus den Radiusmessungen und den Druckmessungen Verwenden einer Funktion umfasst, die Änderungen des Radius des Körperteils mit einer oder mehreren Gewebeeigenschaften und dem auf das Körperteil angewendeten Druck in Beziehung setzt.

9. Verfahren nach einem der Ansprüche 1-8, wobei der Schritt des Bestimmens (107) der Gewebeeigenschaft Beurteilen unter Verwendung der erhaltenen Radiusmessungen, Δ*Rᵢ*, und der erhaltenen Druckmessungen, Δ*pᵢ*, umfasst: $Δ {R}_{i} = \frac{- 1}{{E}_{Arm} \left(Δ{p}_{i}\right)} \frac{{R}_{i 0} \left(1-{υ}_{arm}^{2}\right) \left({R}_{i 0}^{2}-{R}_{b}^{2}\right) Δ {p}_{i}}{\left({R}_{i 0}^{2}\left(1+{υ}_{Arm}\right)+{R}_{b}^{2}\left(1-{υ}_{Arm}\right)\right)}$ mit ${E}_{Arm} \left(Δ{p}_{i}\right) = {E}_{0} + k ⋅ Δ {p}_{i}$ wobei *E*₀ der Elastizitätsmodul des Gewebes im Körperteil ist, *k* ein Parameter ist, der einen nichtlinearen E-Modul des Gewebes beschreibt, *v_{Arm}* die Poisson'sche Zahl des Gewebes ist, *R*_{*i*0} ein anfänglicher Radius des Körperteils ist, wenn kein Druck durch die Schalenmanschetteneinrichtung auf das Körperteil angewendet wird, und *R_{b}* ein Radius eines Knochens im Körperteil ist.

10. Computergestütztes Verfahren zum Bestimmen einer Messung einer physiologischen Eigenschaft eines Subjekts, wobei das Verfahren Folgendes umfasst:
Bestimmen einer Gewebeeigenschaft eines Körperteils des Subjekts gemäß dem Verfahren nach einem der Ansprüche 1-9;
Verwenden der bestimmten Gewebeeigenschaft zum Kalibrieren von Druckmessungen durch das Sensorpad; und
Bestimmen einer Messung der physiologischen Eigenschaft unter Verwendung der kalibrierten Druckmessungen.

11. System (20) zum Bestimmen einer Gewebeeigenschaft eines Körperteils, wobei das System Folgendes umfasst:
eine Schalenmanschetteneinrichtung (22), die so konfiguriert ist, dass sie ein Körperteil mindestens teilweise umschließt und eine Vielzahl von unterschiedlichen Drücken auf das Körperteil anwendet, wobei die Schalenmanschetteneinrichtung (22) einen Hartschalenabschnitt (28) umfasst, der so konfiguriert ist, dass er um mindestens einen Abschnitt des Körperteils platziert werden kann, und eine aufblasbare Manschette (26), die den Hartschalenabschnitt (28) umgibt, die so konfiguriert ist, dass sie aufgeblasen und/oder entlüftet werden kann, um über den Hartschalenabschnitts (28) die Vielzahl von unterschiedlichen Drücken auf das Körperteil anzuwenden;
einen oder mehrere Sensoren (34, 36) zum Bereitstellen von Radiusmessungen eines Radius des Körperteils oder von Änderungen des Radius des Körperteils, wenn die Vielzahl von verschiedenen Drücken auf das Körperteil angewendet wird, und von Druckmessungen der auf den Körperteil angewendeten verschiedenen Drücke, wobei
- zum Bereitstellen der Radiusmessungen die Sensoren (34, 36) einen Radiussensor (34) oder ein Sensorpad (36), das an der Innenseite des Hartschalenabschnitts positioniert ist, einschließen und
- zum Bereitstellen der Druckmessung die Sensoren (34, 36) das Sensorpad (36) oder einen Manschettendrucksensor einschließen; und
eine Vorrichtung (24), die konfiguriert ist, um die Gewebeeigenschaften des Körperteils zu bestimmen, wobei die Vorrichtung eine Verarbeitungseinheit (38) umfasst, die so konfiguriert ist, dass sie das Verfahren nach einem der Ansprüche 1-9 durchführt.

12. System (20) zum Bestimmen einer Messung einer physiologischen Eigenschaft eines Subjekts, wobei das System Folgendes umfasst:
eine Schalenmanschetteneinrichtung (22), die so konfiguriert ist, dass sie ein Körperteil mindestens teilweise umschließt und eine Vielzahl von unterschiedlichen Drücken auf das Körperteil anwendet, wobei die Schalenmanschetteneinrichtung (22) einen Hartschalenabschnitt (28) umfasst, der so konfiguriert ist, dass er um mindestens einen Abschnitt des Körperteils platziert werden kann, und eine aufblasbare Manschette (26), die den Hartschalenabschnitt (28) umgibt, die so konfiguriert ist, dass sie aufgeblasen und/oder entlüftet werden kann, um über den Hartschalenabschnitts (28) die Vielzahl von unterschiedlichen Drücken auf das Körperteil anzuwenden;
einen oder mehrere Sensoren (34, 36) zum Bereitstellen von Radiusmessungen eines Radius des Körperteils oder von Änderungen des Radius des Körperteils, wenn die Vielzahl von verschiedenen Drücken auf das Körperteil angewendet wird, und von Druckmessungen der auf den Körperteil angewendeten verschiedenen Drücke, wobei
- zum Bereitstellen der Radiusmessungen die Sensoren (34, 36) einen Radiussensor (34) oder ein Sensorpad (36), das an der Innenseite des Hartschalenabschnitts positioniert ist, einschließen und
- zum Bereitstellen der Druckmessung die Sensoren (34, 36) das Sensorpad (36) oder einen Manschettendrucksensor einschließen; und
eine Vorrichtung (24), die konfiguriert ist, um die Messung der physiologischen Merkmale des Probanden zu bestimmen, wobei die Vorrichtung eine Verarbeitungseinheit (38) umfasst, die zur Durchführung des Verfahrens nach Anspruch 10 konfiguriert ist.

13. Computerprogrammprodukt, das Anweisungen umfasst, um zu bewirken, dass das System nach Anspruch 11 das Verfahren nach einem der Ansprüche 1-9 ausführt.

14. Computerprogrammprodukt, das Anweisungen umfasst, um zu bewirken, dass das System nach Anspruch 12 das Verfahren nach Anspruch 10 ausführt.

## Revendications

1. Procédé informatisé de détermination d'une propriété tissulaire d'une partie du corps, le procédé comprenant :
la commande (101) d'un appareil à manchette à coque (22) pour appliquer une pluralité de pressions différentes à la partie du corps, dans lequel l'appareil à manchette à coque enveloppe au moins partiellement la partie du corps, et dans lequel l'appareil à manchette à coque comprend une partie à coque rigide placée au moins partiellement autour de la partie du corps, et une manchette gonflable entourant la partie à coque rigide qui est gonflée et/ou dégonflée pour appliquer la pluralité de pressions différentes à la partie du corps via la partie à coque rigide ;
l'obtention (103) de mesures de rayon d'un rayon de la partie du corps, ou des changements dans le rayon de la partie du corps, lorsque la pluralité de pressions différentes sont appliquées à la partie du corps, à l'aide d'un capteur de rayon (34) ou à l'aide de mesures provenant d'un coussinet de capteur (36) positionné à l'intérieur de la partie de la coque rigide ;
l'obtention (105) de mesures de pression des différentes pressions appliquées à la partie du corps,
dans lequel les mesures de pression sont obtenues à l'aide du capteur, ou
dans lequel les mesures de pression sont obtenues à l'aide d'un capteur de pression de brassard permettant de mesurer la pression dans le brassard gonflable au fur et à mesure que celui-ci est gonflé et/ou dégonflé et de déterminer les mesures de pression à partir de la mesure d'un capteur de pression de brassard respectif et d'une mesure de rayon correspondante ; et
la détermination (107) de la propriété tissulaire de la partie du corps à partir des mesures du rayon et des mesures de pression.

2. Procédé selon la revendication 1, dans lequel l'étape de détermination des mesures de pression comprend :
la détermination d'une mesure de pression en fonction de la mesure d'un capteur de pression du brassard, d'une mesure de rayon correspondante, d'un coefficient de frottement de la partie rigide de la coque et d'une longueur totale de la partie rigide de la coque.

3. Procédé selon la revendication 1, dans lequel le capteur de rayon est un capteur configuré pour mesurer la déformation de la partie de la coque rigide.

4. Procédé selon la revendication 1 ou 3, dans lequel le capteur de rayon est configuré ou agencé de telle sorte que la déformation de la partie de la coque rigide modifie une ou plusieurs propriétés électriques du capteur de rayon.

5. Procédé selon l'une quelconque des revendications 1, 3 ou 4, dans lequel le capteur de rayon est une jauge de contrainte.

6. Procédé selon la revendication 1 ou 2, dans lequel les mesures du rayon sont obtenues à partir de mesures de la pression dans le brassard gonflable et de mesures du débit de fluide entrant et sortant du brassard gonflable.

7. Procédé selon la revendication 1, dans lequel les mesures de rayon sont obtenues à partir de mesures de la pression dans le brassard gonflable, de mesures de pression obtenues à l'aide du capteur, d'un facteur de friction pour la partie de la coque rigide et de la longueur de la partie de la coque rigide.

8. Procédé selon l'une quelconque des revendications 1 à 7, dans lequel l'étape de détermination (107) de la propriété tissulaire de la partie du corps à partir des mesures de rayon et des mesures de pression comprend l'utilisation d'une fonction qui relie les changements de rayon de la partie du corps à une ou plusieurs propriétés tissulaires et à la pression appliquée à la partie du corps.

9. Procédé selon l'une quelconque des revendications 1-8, dans lequel l'étape de détermination (107) de la propriété tissulaire comprend l'évaluation, à l'aide des mesures de rayon obtenues, Δ*Rᵢ* et des mesures de pression obtenues, Δ*pᵢ* de : $Δ {R}_{i} = \frac{- 1}{{E}_{bras} \left(Δ{p}_{i}\right)} \frac{{R}_{i 0} \left(1-{υ}_{bras}^{2}\right) \left({R}_{i 0}^{2}-{R}_{b}^{2}\right) Δ {p}_{i}}{\left({R}_{i 0}^{2}\left(1+{υ}_{bras}\right)+{R}_{b}^{2}\left(1-{υ}_{bras}\right)\right)}$ avec ${E}_{bras} \left(Δ{p}_{i}\right) = {E}_{0} + k ⋅ Δ {p}_{i}$ dans lequel *E*₀ est le module d'élasticité du tissu dans la partie du corps, *k* est un paramètre décrivant un module E non linéaire du tissu, *v_{bras}* est le coefficient de Poisson du tissu, *R*_{*i*0} est un rayon initial de la partie du corps lorsqu'aucune pression n'est appliquée à la partie du corps par l'appareil de manchette à coque, et *R_{b}* est un rayon d'un os dans la partie du corps.

10. Procédé informatisé de détermination de la mesure d'une caractéristique physiologique d'un sujet, le procédé comprenant :
la détermination d'une propriété tissulaire d'une partie du corps du sujet selon le procédé selon l'une quelconque des revendications 1-9 ;
l'utilisation de la propriété tissulaire déterminée pour calibrer les mesures de pression effectuées par le capteur ; et
la détermination d'une mesure de la caractéristique physiologique à l'aide des mesures de pression étalonnées.

11. Système (20) pour déterminer une propriété tissulaire d'une partie du corps, le système comprenant :
un appareil à manchette à coque (22) configuré pour envelopper au moins partiellement une partie du corps et pour appliquer une pluralité de pressions différentes à la partie du corps, dans lequel l'appareil à manchette à coque (22) comprend une partie à coque rigide (28) configurée pour être placée autour d'au moins une partie de la partie du corps et une manchette gonflable (26) entourant la partie à coque rigide (28) qui est configurée pour être gonflée et/ou dégonflée pour appliquer la pluralité de pressions différentes à la partie du corps via la partie à coque rigide (28) ;
un ou plusieurs capteurs (34, 36) pour fournir des mesures du rayon d'une partie du corps, ou des changements dans le rayon de la partie du corps, lorsque plusieurs pressions différentes sont appliquées à la partie du corps, et des mesures de pression des différentes pressions appliquées à la partie du corps, dans lequel
- pour fournir les mesures de rayon, les capteurs (34, 36) incluent un capteur de rayon (34) ou un capteur (36) positionné à l'intérieur de la partie rigide de la coque, et
- pour fournir les mesures de pression, les capteurs (34, 36) incluent le coussinet capteur (36) ou un capteur de pression de brassard ; et
un dispositif (24) configuré pour déterminer la propriété tissulaire de la partie du corps, dans lequel le dispositif comprend une unité de traitement (38) configurée pour mettre en œuvre le procédé selon l'une quelconque des revendications 1-9.

12. Système (20) pour déterminer la mesure d'une caractéristique physiologique d'un sujet, ce système comprenant :
un appareil à manchette à coque (22) configuré pour envelopper au moins partiellement une partie du corps et pour appliquer une pluralité de pressions différentes à la partie du corps, dans lequel l'appareil à manchette à coque (22) comprend une partie à coque rigide (28) configurée pour être placée autour d'au moins une partie de la partie du corps et une manchette gonflable (26) entourant la partie à coque rigide (28) qui est configurée pour être gonflée et/ou dégonflée pour appliquer la pluralité de pressions différentes à la partie du corps via la partie à coque rigide (28) ;
un ou plusieurs capteurs (34, 36) pour fournir des mesures du rayon d'une partie du corps, ou des changements dans le rayon de la partie du corps, lorsque plusieurs pressions différentes sont appliquées à la partie du corps, et des mesures de pression des différentes pressions appliquées à la partie du corps, dans lequel
- pour fournir les mesures de rayon, les capteurs (34, 36) incluent un capteur de rayon (34) ou un capteur (36) positionné à l'intérieur de la partie rigide de la coque, et
- pour fournir les mesures de pression, les capteurs (34, 36) incluent le coussinet capteur (36) ou un capteur de pression de brassard ; et
un dispositif (24) configuré pour déterminer la mesure de la caractéristique physiologique du sujet, dans lequel le dispositif comprend une unité de traitement (38) configurée pour mettre en œuvre le procédé selon la revendication 10.

13. Produit de programme informatique comprenant des instructions pour faire en sorte que le système selon la revendication 11 exécute le procédé selon l'une quelconque des revendications 1-9.

14. Produit de programme informatique comprenant des instructions pour amener le système selon la revendication 12 à exécuter le procédé selon la revendication 10.
